# EUROPÄISCHE PATENTSCHRIFT

(11) **EP 0 861 068 B1**
(45) Veröffentlichungstag und Bekanntmachung des Hinweises auf die Patenterteilung: **14.08.2002**
(21) Anmeldenummer: 96938066.6
(22) Anmeldetag: 04.11.1996
(51) Int. Cl.: A61K 9/127

(54) **VERFAHREN UND VORRICHTUNG ZUR HERSTELLUNG EINER PARENTERALEN ARZNEISTOFF-ZUBEREITUNG**
METHOD AND DEVICE FOR PRODUCING A PARENTERAL MEDICAMENT
PROCEDE ET DISPOSITIF POUR LA PRODUCTION D'UNE PREPARATION MEDICAMENTEUSE ADMINISTREE PAR VOIE PARENTERALE

(30) Priorität: 15.11.1995 DE 19542499
(43) Veröffentlichungstag der Anmeldung: 02.09.1998
(73) Patentinhaber: BAYER AG, 51368 Leverkusen (DE)
(72) Erfinder: KLINKSIEK, Bernd, D-51429 Bergisch Gladbach (DE); MAHIOUT, Said, D-51467 Bergisch Gladbach (DE); NOTHELLE, Ricarda-Christine, D-51371 Leverkusen (DE); HAMANN, Hans-Jürgen, D-41549 Dormagen (DE); SDEBIK, Jürgen, D-40477 Düsseldorf (DE)
(86) Internationale Anmeldenummer: EP9604795
(87) Internationale Veröffentlichungsnummer: WO9717946

(56) Entgegenhaltungen:
- EP-A- 0 560 138
- WO-A-83/00089
- WO-A-94/08626

## Beschreibung

Die Erfindung betrifft ein Verfahren zur Herstellung einer Formulierung für parenteral applizierbare Arzneistoff-Zubereitungen mit einer Liposomen-Dispersion als Träger für den pharmazeutischen Wirkstoff, wobei zur Herstellung der Liposomen-Dispersion eine wäßrige Vordispersion amphiphiler Stoffe einem Hochdruck-Homogenisator zugeführt wird. Ein derartiges Verfahren wird prinzipiell in DE 42 07 481 beschrieben.

Zur Herstellung von Liposomen ist eine Vielzahl von Verfahren beschrieben worden (s. z.B. Arndt, "Liposomen", Akademie-Verlag Berlin, 1986). Gegenstand dieser Arbeiten sind häufig Experimente im Labormaßstab. Als üblicher Startprozeß ist dabei das Auflösen von Phospholipiden in organischen Lösungsmitteln vorgesehen, die im Verlauf des weiteren Herstellungsprozesses vor der Homogenisierung wieder entfernt werden (DE 35 15 335).

Bei dem Direktdispergierverfahren gemäß DE 42 07 481 werden das Phospholipid und der kristalline Wirkstoff direkt in Wasser dispergiert. Nach dem Quellen der Phospholipide in Wasser entstehen zunächst grobverteilte Liposomen, die mechanisch zerkleinert werden müssen. Der Wirkstoff lagert sich dabei an die entstehenden Lipid-Doppelschichten der entstehenden Liposomen ein bzw. an. Da viele Liposomen-Formulierungen nicht hitzesterilisierbar sind (Teilchenaggregation, Phospholipidhydrolyse), ist eine Zerkleinerung der Liposomen durch Hochdruck-Homogenisation erforderlich, bis die liposomalen Dispersionen sterilfiltrierbar (Teilchengröße < 200 nm) sind.

Die Zerkleinerung geschieht dabei in zwei Schritten :
a) Zunächst wird die Liposomen-Dispersion mit einer hochtourigen Rotor/Stator-Maschine auf Partikelgrößen von 500 bis 5.000 µm zerkleinert.
b) Anschließend erfolgt eine Feinzerkleinerung auf Partikelgrößen von 40 bis 100 nm mit an sich bekannten Hochdruck-Homogenisatoren.

Der Erfindung liegt die Aufgabe zugrunde, ein Verfahren zur Herstellung von Liposomen mit feinst möglicher Partikelgröße für parenterale Anwendungen in reproduzierbarer Produktqualität zu entwickeln. Die Liposomen-Dispersion soll dabei einerseits ohne Filterrückstand sterilfiltrierbar und andererseits so fein sein, daß der Wirkstoff bei der parenteralen Anwendung die feinsten Gefäßverästelungen der Blutbahnen passieren kann.

Diese Aufgabe wird erfindungsgemäß dadurch gelöst, daß eine wäßrige Vordispersion ampiphiler Substanzen unter einem Druck von 500 bar bis 900 bar, vorzugsweise 700 bar bis 800 bar, durch eine zylindrische Homogenisierdüse mit einem Durchmesser von 0,1 mm bis 0,5 mm, vorzugsweise 0,1 mm bis 0,2 mm, gepumpt wird. Unter diesen Bedingungen können extrem feinteilige Dispersionen mit mittleren Partikelgrößen von 30 nm bis 100 nm erreicht werden.

Geeignete ampiphile Substanzen sind insbesondere Phospholipide, Cholesterinderivate und synthetische Amphiphile.

Zur Herstellung der Vordispersion wird eine aus der wäßrigen, amphiphile Substanzen enthaltenden Dispersion und dem pharmazeutischen Wirkstoff bestehende Primärdispersion durch eine gröbere zylindrische Homogenisierdüse mit einem Durchmesser von 0,3 mm bis 0,7 mm gepumpt. Durch diese Art der Vorhomogenisierung können die bei den bisher zur Vordispergierung häufig verwendeten Rotor/Stator-Maschinen auftretenden Partikelkontaminationen vermieden werden.

Gemäß einer bevorzugten Ausführung wird die Vordispersion im Kreislauf so lange durch die Homogenisierdüse gepumpt, bis die mittlere Teilchengröße der Liposomen-Dispersion im Bereich zwischen 35 nm und 80 nm mit einer Standardabweichung von 3 nm bis 7 nm liegt. Diese Teilchen-Eigenschaften werden insbesondere dann erreicht, wenn man die Vordispersion im Kreislauf 10- bis 30-mal durch die Homogenisierdüse strömen läßt.

Besonders gute Ergebnisse werden erzielt, wenn die Dispersion im Homogenisierungskreislauf durch einen der Homogenisierdüse vorgeschalteten Wärmetauscher auf eine Temperatur im Bereich von 50°C bis 70°C aufgeheizt wird.

Vorzugsweise wird der Vorratsbehälter auf einem niedrigeren Temperaturniveau gehalten und die Dispersion erst unmittelbar vor der Düse aufgeheizt.

Da in der Homogenisierdüse eine Energiedissipation und damit eine Erwärmung stattfindet, ist es zweckmäßig, die Dispersion unmittelbar nach der Homogenisierdüse durch einen nachgeschalteten Wärmetauscher wieder auf Temperaturen im Bereich von 50°C bis 70°C abzukühlen.

Zum Umpumpen der Liposomen-Dispersion wird vorteilhaft eine Hochdruck-Membrankolbenpumpe verwendet, die den Vorteil besitzt, daß kein Abrieb und kein Schmiermittel in den Pumpenraum gelangen kann, und die damit als Kontaminationsquelle ausscheidet.

Die Vorrichtung zur Durchführung des Verfahrens besteht aus mindestens einer Homogenisierdüse mit einem Zufluß- und einem Abflußkanal und ist dadurch gekennzeichnet, daß die Homogenisierdüse aus einer in einen Stahlkörper eingepreßten hartkeramischen Platte mit einer Bohrung von 0,1 mm bis 0,5 mm, vorzugsweise 0,1 mm bis 0,2 mm, besteht und daß der Zuflußkanal zu der Bohrung und der Abflußkanal von der Bohrung ebenfalls in den Stahlkörper eingearbeitet sind.

Um hohe Düsenstandzeiten zu erzielen, werden vorteilhaft hartkeramische Platten aus Zirkonoxid oder Siliziumkarbid in den Stahlkörper eingepreßt.

Gemäß einer Weiterentwicklung der Erfindung weist der Stahlkörper mehrere, einander paarweise gegenüberstehende Homogenisierdüsen auf, wobei die Zuflußkanäle der Homogenisierdüsen parallel geschaltet sind und die Abflußkanäle im Stahlkörper in einen gemeinsamen Sammelkanal münden. Diese Ausführung hat sich insbesondere bei hohen Durchsätzen gut bewährt. Dabei kann eine genaue Maßstabsübertragung (scale up) erreicht werden.

Mit der Erfindung werden folgende Vorteile erzielt:
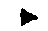 Herstellung und Bereitstellung stabiler, liposomaler Formulierungen insbesondere mit in Wasser schwerlöslichen Wirkstoffen
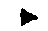 Erzielung einer engen Teilchengrößenverteilung mit hoher Reproduzierbarkeit
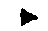 Schonende Behandlung insbesondere von temperaturempfindlichen Wirkstoffen
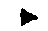 Einwandfreie und genaue Maßstaosübertragung durch modularen Aufbau der Düsenhomogenisiervorrichtung
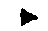 Reduzierung von Kontaminationsquellen durch Minimierung des Düsenabriebs und Einsatz einer Hochdruck-Membrankolbenpumpe
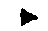 Gute Reinigungsmöglichkeiten aufgrund einer spalt- und totraumarmen Konstruktion
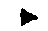 Verbesserte Standzeiten der Anlage.

Im Folgenden werden Ausführungsbeispiele der Erfindung an Hand von Zeichnungen näher erläutert. Es zeigen:
- Fig. 1: ein Fließschema der Anlage
- Fig. 2: den Aufbau einer Homogenisierdüse
- Fig. 3: eine Seitenansicht eines aus mehreren parallel geschalteten Einzeldüsen bestehenden Düsenhomogenisators
- Fig. 4: einen Querschnitt durch den Düsenhomogenisator nach Fig. 3 und
- Fig. 5: eine grafische Darstellung der Ergebnisse bei dem Ausführungsbeispiel Nr. 2

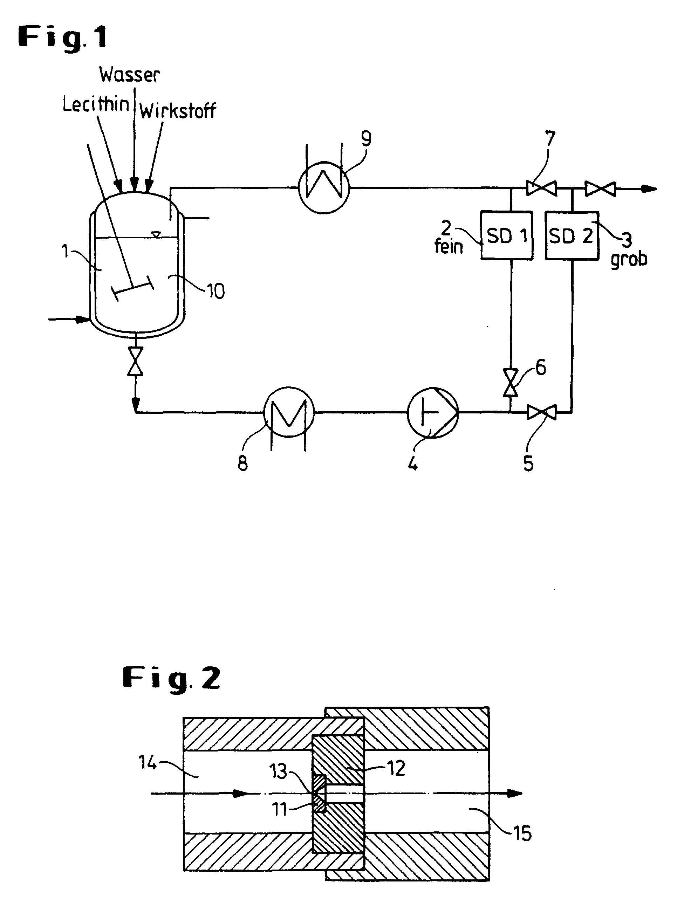

Gemäß Fig. 1 wird zunächst eine Lecithin/Wasser-Rohdispersion durch Rühren in dem heiz- und kühlbaren Rührkessel 1 hergestellt und bei Temperaturen von 50°C bis 70°C und Drücken von 500 bis 1000 bar durch die Homogenisierdüse 2 mit einem Durchmesser von 0,2 mm oder durch die Homogenisierdüse 3 mit einem Durchmesser von 0,5 mm bei Drücken von 40 bis 200 bar mittels einer Hochdruck-Membrankolbenpumpe 4 gepumpt. Die Umschaltung auf die gewünschte Düse 2 oder 3 erfolgt mittels der Ventile 5, 6 und 7. Da die Homogenisierung bei 50°C bis 70°C günstiger abläuft, wird die Mischung im Wärmetauscher 8 vorher aufgeheizt und im Wärmetauscher 9 wieder abgekühlt, um temperaturbedingte Produktschädigungen, z. B. durch Hydrolyse, zu vermeiden. Diese Abkühlung ist erforderlich, da die Dispersion beim Durchgang durch die Homogenisierdüse weiter aufgeheizt wird. Die Temperatur im Rührkessel 1 wird auf einem niedrigeren Niveau (≦ 50°C) gehalten, um Produktschädigungen zu minimieren. Danach wird der Wirkstoff zugegeben und mit dem Rührer 10 vordispergiert. Die so hergestellte Primärdispersion wird dann durch die Homogenisierdüse 3 mit dem gröberen Durchmesser durch 5-maliges Umpumpen vordispergiert und anschließend im Kreislauf über die Wärmetauscher 8 und 9 und die feinere Homogenisierdüse 2 feindispergiert. Wenn z.B. nach 20 Umläufen bei Homogenisierdrücken von 500 bis 800 bar eine mittlere Partikelgröße von 35 bis 60 nm erreicht ist, wird die Liposomen-Formulierung bei voller Kühlung abgepumpt und sterilfiltriert.

Zur Herstellung der Vordispergierung ist es auch möglich, daß alle drei Komponenten Wasser, Phospholipid und Wirkstoff im Rührkessel 1 vordispergiert und anschließend in der Düse 3 vorzerkleinert werden, bevor über die Düse 2 feinhomogenisiert wird.

Den detailgenauen Aufbau einer Homogenisierdüse zeigt Fig. 2. Sie besteht aus einer hartkeramischen Platte 11, z.B. aus Zirkonoxid, die in eine Stahlplatte 12 eingepreßt ist, und in der Mitte eine zylindrische Düsenbohrung 13 von ca. 0,2 mm Durchmesser aufweist. Die Düsenbohrung 13 erweitert sich trichterförmig in Strömungsrichtung. Der Zufluß 14 für die Dispersion liegt vor der Düsenbohrung 13. Der Abfluß 15 schließt sich an die trichterförmige Erweiterung an. Wichtig ist bei dieser Ausführungsform, daß keine gegenüberliegenden Wandflächen vorhanden sind, die von dem austretenden Strahl hinter der Düse zerstört werden können. Aus diesem Grund ist eine Anordnung vorzuziehen, bei der sich die Düsen einander paarweise gegenüberstehen, so daß die Impulse der Flüssigkeitsstrahlen sich gegenseitig aufheben und die noch vorhandene Restenergie zur Zerkleinerung genutzt werden kann.

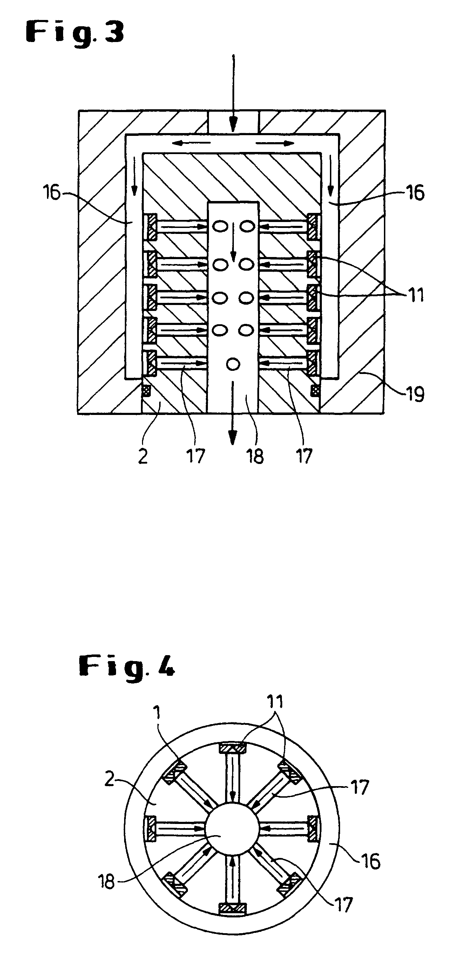

Die Fig. 3 und 4 zeigen eine Ausführungform eines Düsenhomogenisators für hohe Durchsätze mit vielen parallel geschalteten, paarweise einander gegenüberstehenden Düsenbohrungen in einer sternförmigen, rotationssysmmetrischen Anordnung (s. Fig. 4), die mit einer gemeinsamen Ringraum-Zuleitung 16 verbunden sind. Die zu dispergierende Flüssigkeit wird durch die Ringraumleitung 16 zugeführt und strömt nach der Dispergierung bzw. Homogenisierung durch die Abflußkanäle 17 in einen zentralen gemeinsamen Sammelkanal 18. Sämtliche Bauteile 11, 16, 17 und 18 sind in einen zylindrischen Stahlblock 19 eingearbeitet.

Die Keramikplatten 11 mit der Düsenbohrung 13 und der trichterförmigen Erweiterung (s. Fig. 2) werden vor dem Einpressen in den Stahlkörper 12 maßgenau gefertigt. Der Bohrungsdurchmesser beträgt dabei 0,1 bis 0,5 mm und das Längen/Durchmesser-Verhältnis 1,5 bis 2. Wie in Fig. 3 und 4 dargestellt, erweitert sich die Bohrung auf den Durchmesser des Abflußkanals 17.

Neben der kreisrunden Bohrung in der Keramikscheibe sind auch Düsenbohrungen mit unrunden Querschnitten, wie Elipsen oder Schlitze, möglich. Denkbar wäre auch eine Konstruktion aus Vollkeramik. Im Hinblick auf die Aufgabenstellung einer reproduzierbaren Produktqualität bei feinst möglicher Partikelgröße und geringst möglichem Verschleiß sind jedoch Stahl/Keramik-Verbundkonstuktionen am günstigsten, da wesentlich höhere Fertigungsgenauigkeiten ereicht werden, was insbesondere für sehr hohe Durchsätze und bei vielen parallel geschalteten Düsen gilt.

### Ausführungsbeispiele

### Beispiel 1:

Die Anlage nach Fig. 1 ist mit einem Rührkessel 1 mit einem Volumen von 6 l, einer Dreifachmembranpumpe 4, die bei einem Durchsatz von 60 l/h max. 900 bar erreichen kann, und mit Düsenhomogenisatoren nach Fig. 3 und 4 mit zwei gegenüberliegenden Bohrungen von 0,2 mm für die Düse 2 (SD 1) und mit zwei gegenüberliegenden Bohrungen von 0,5 mm Durchmesser für die Düse 3 (SD 2) ausgerüstet.

Im Rührkessel 1 werden 5,59 kg destilliertes Wasser 30 min mit Stickstoff begast. Nach der Begasung werden 16,2 g Natriumascorbat im Wasser gelöst und anschließend 580,5 g gereinigtes Eilicithin (Phoshatidylcholin > 94 %) zugegeben. Diese Mischung wird 30 min mit dem schnellaufenden Rührer 10 bei 65°C dispergiert. Die Dispersion wird danach in fünf Durchgängen bei 800 bar und 65°C (Kesseltemperatur 50°C) über die Düse 2 homogenisiert. Danach werden 145 g des Wirkstoffes 2-Amino-1,4-dihydro-5-cyano-6-methyl-4-(3-phenylchinolin-5-yl)pyridin-3-carbonsäureisopropylester zugesetzt und bei einem Druck von 25 bar durch die Düse 3 vorhomogenisiert und ebenfalls 5-mal umgepumpt. Anschließend werden 20 Durchgänge bei 65°C und 800 bar Vordruck über die Düse 2 homogenisiert. Die mittlere Partikelgröße beträgt 48 nm mit einer Standardabweichung von 5 nm. Die Dispersion kann problemlos sterilfiltriert werden.

### Beispiel 2:

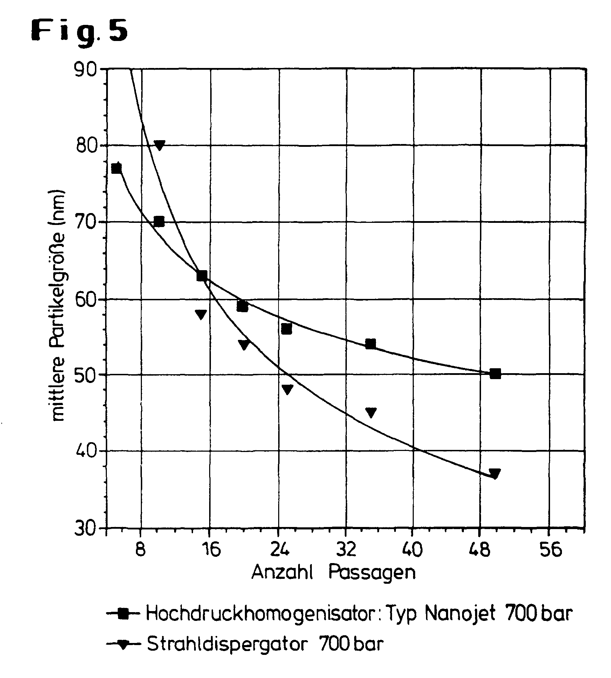

Nach Beispiel 1 werden Ansätze bei 700 bar Homogenisierdruck mit einer unterschiedlichen Anzahl von Durchläufen hergestellt. Die Ergebnisse sind in Fig. 5 grafisch dargestellt und mit 1 l Ansätzen gleicher Rezeptur, die in einem Laborhochdruck-Homogenisator vom Typ Nanojet hergestellt wurden, verglichen. Trotz gröberer Ausgangsteilchengröße werden mit dem erfindungsgemäßen Verfahren bei gleichen Homogenisierdrücken und gleicher Anzahl von Durchläufen wesentlich feinere Partikelgrößen erreicht.

### Beispiel 3:

800 g Wasser für Injektionszwecke werden im Rührkessel 1 10 min mit Stickstoff begast. Anschließend werden in dieser Wassermenge 1 g Ascorbinsäure, 150 g Glucose und 1 g L-Arginin gelöst. In diesem Medium werden 1,667 g Nimodipin und 83,35 g hochreines Ei-Phospholipid (z.B. Lipoid EPC) dispergiert und mit stickstoffbegastem Wasser auf 1055,5 g aufgefüllt

Diese Dispersion wird 30 min bei 75°C unter Stickstoffschutz mit einem schnellaufendem Rührwerk, z.B. Ultra-Turrax, vorhomogenisiert. Anschließend wird bei 75°C und 800 bar mit einer Vorrichtung nach Beispiel 1 mit zwei gegenüberliegenden Saphirdüsen von 0,2 mm Durchmessser und mit scharfkantigem Ein- und Auslauf und einer Dicke der Saphirscheibe von 0,4 mm auf 45 nm homogenisiert.

Die Dispersion wird abgekühlt und nach Sterilfiltration in 250 ml- oder 50 ml-Flaschen abgefüllt und gefriergetrocknet.

Das Lyophilisat kann mit isotonischer Glucoselösung ad 250 ml oder mit Aqua p.i. ad 50 ml rekonstituiert werden. Die Partikelgröße beträgt 50 nm mit einer Standardabweichung von 7 nm.

## Patentansprüche

1. Verfahren zur Herstellung einer Formulierung für parenteral applizierbare Arzneistoff ubereitungen mit einer Liposomen-Dispersion als Träger für einen pharmazeutischen Wirkstoff, wobei zur Herstellung der Liposomen-Dispersion eine wäßrige Vordispersion einer oder mehrerer amphiphiler Stoffe einem Hochdruck-Homogenisator zugeführt wird, **dadurch gekennzeichnet, daß** die Vordispersion unter einem Druck von 600 bar bis 900 bar durch eine Homogenisierdrüse (2) mit einem Durchmesser von 0,1 bis 0,5 mm gepumpt wird, und zur Herstellung der Vordispersion eine diamphiphilen Stoffe und den pharmazeutischen Wirkstoff enthaltende wässrige Primärdispersion durch eine Homogenisierdrüse (3) mit einem Durchmesser von 0,3 bis 0,7 mm gepumpt wird.

2. Verfahren nach **Anspruch** 1, **dadurch gekennzeichnet, daß** die Vordispersion im Kreislauf so lange durch die Homogenisierdüse (13) gepumpt wird, bis die mittlere Teilchengröße der Liposomen-Dispersion im Bereich zwischen 35 nm und 80 nm mit einer Standardabweichung von 4 nm bis 8 nm liegt.

3. Verfahren nach Anspruch 1 oder 2 **dadurch gekennzeichnet, daß** die Vordispersion 10-mal bis 30-mal im Kreislauf durch die Homogenisierdrüse (2) strömt.

4. Verfahren nach den Ansprüchen 1 bis 3 **dadurch gekennzeichnet, daß** die Vordispersion durch einen der Homogenisierdüse (2) vorgeschalteten Wärmetauscher (8) auf eine Temperatur im Bereich von 50°C bis 70°C aufgeheizt wird.

5. Verfahren nach den Ansprüchen 1 bis 4 **dadurch gekennzeichnet, daß** die Vordispersion im Rührkessel (1) auf einer Temperatur ≦50°C gehalten wird.

6. Verfahren nach den Ansprüchen 1 bis 5, **dadurch gekennzeichnet, daß** die in der Homogenisierdüse (2) erhitzte Dispersion durch einen der Homogenisierdüse (2) nachgeschalteten Wärmetauscher (9) auf eine Temperatur im Bereich von 50°C bis 70°C gekühlt wird.

7. Verfahren nach den Ansprüchenl bis 6, **dadurch gekennzeichnet, daß** als Pumpe eine Membranpumpe (4) verwendet wird.

8. Vorrichtung zur Durchführung des Verfahrens nach den Ansprüchen 1 bis 7 ausgehend von mindestens einer Homogenisierdüse mit einem Zuflußund einem Abflußkanal, **dadurch gekennzeichnet, daß** die Homogenisierdüse aus einer in einen Stahlkörper (12, 19) eingepreßten hartkeramischen Platte (11) mit einer Bohrung (13) von 0,1 mm bis 0,5 mm, vorzugsweise 0,1 mm bis 0,2 mm, besteht und daß der Zuflußkanal (14, 16) zu der Bohrung (13) und der Abflußkanal (15, 18) von der Bohrung (13) ebenfalls in den Stahlkörper (12, 19) eingearbeitet sind.

9. Vorrichtung nach Anspruch 8 **dadurch gekennzeichnet, daß** die hartkeramische Platte (11) aus Zirkonoxid oder Siliziumkarbid besteht.

10. Vorrichtung nach den Ansprüchen 8 bis 9, **dadurch gekennzeichnet, daß** der Stahlkörper (19) mehrere, einander paarweise gegenüberstehende Homogenisierdüsen mit parallel geschalteten Zuflußkanälen (16) aufweist und daß die Abflußkanäle (17) im Stahlkörper (19) in einen gemeinsamen Sammelkanal (18) münden.

## Claims

1. Procedure for the preparation of a formulation for parenterally administrable pharmaceutical preparations having a liposome dispersion as a carrier for a pharmaceutical active compound, where for the preparation of the liposome dispersion an aqueous predispersion of one or more amphiphilic substances is fed to a high-pressure homogenizer, **characterized in that** the predispersion is pumped under a pressure from 600 bar to 900 bar, through a homogenizer nozzle (2) having a diameter of 0.1 to 0.5 mm, for the preparation of the predispersion an aqueous primary dispersion containing the amphiphilic substances and the pharmaceutical active compound is pumped through a homogenizer nozzle (3) having a diameter of 0.3 to 0.7 mm.

2. Procedure according to Claim 1, **characterized in that** the predispersion is recirculated through the homogenizer nozzle (13) by pumping until the average particle size of the liposome dispersion is in the range between 35 nm and 80 nm with a standard deviation of 4 nm to 8 nm.

3. Procedure according to Claim 1 or 2, **characterized in that** the predispersion flows through the homogenizer nozzle (2) 10 times to 30 times in circulation.

4. Procedure according to Claims 1 to 3, **characterized in that** the predispersion is heated to a temperature in the range from 50°C to 70°C by a heat exchanger (8) connected before the homogenizer nozzle (2).

5. Procedure according to Claims 1 to 4, **characterized in that** the predispersion in the stirring vessel (1) is kept at a temperature of ≤ 50°C.

6. Procedure according to Claims 1 to 5, **characterized in that** the dispersion heated in the homogenizer nozzle (2) is cooled to a temperature in the range from 50°C to 70°C by a heat exchanger (9) connected after the homogenzier nozzle (2).

7. Procedure according to Claims 1 to 6, **characterized in that** the pump used is a diaphragm-type pump (4).

8. Device for carrying out the procedure according to Claims 1 to 7, starting from at least one homogenizer nozzle having an inlet and an outlet channel, **characterized in that** the homogenizer nozzle consists of a hard ceramic plate (11) with a bore (13) of 0.1 mm to 0.5 mm, preferably 0.1 mm to 0.2 mm, pressed into a steel body (12, 19) and that the inlet channel (14, 16) to the bore (13) and the outlet channel (15, 18) from the bore (13) are likewise incorporated into the steel body (12, 19).

9. Device according to Claim 8, **characterized in that** the hard ceramic plate (11) consists of zirconium oxide or silicon carbide.

10. Device according to Claims 8 or 9, **characterized in that** the steel body (19) has several homogenizer nozzles facing one another in pairs with inlet channels (16) connected in parallel and that the outlet channels (17) in the steel body (19) open into a common collection channel (18).

## Revendications

1. Procédé de production d'une formulation pour préparations médicamenteuses applicables par voie parentérale avec une dispersion de liposomes comme support pour un principe actif pharmaceutique où, pour la production de la dispersion de liposomes, une prédispersion aqueuse d'une ou plusieurs substances amphiphiles est envoyée un homogénéisateur à haute pression, **caractérisé en ce que** la prédispersion est pompée sous une pression de 600 bar à 900 bar au travers d'une buse d'homogénéisation (2) d'un diamètre de 0,1 à 0,5 mm, et pour la production de la prédispersion, une dispersion primaire aqueuse contenant des substances diamphiphiles et le principe actif pharmaceutique est pompée au travers d'une buse d'homogénéisation (3) d'un diamètre de 0,3 à 0,7 mm.

2. Procédé selon la revendication 1, **caractérisé en ce que** la prédispersion est pompée dans le circuit au travers de la buse d'homogénéisation (13) jusqu'à ce que la taille de particules moyenne de la dispersion de liposomes soit située dans le domaine de 35 nm à 80 nm avec un écart-type de 4 nm à 8 nm.

3. Procédé selon la revendication 1 ou 2, **caractérisé en ce que** la prédispersion traverse 10 à 20 fois dans le circuit la buse d'homogénéisation (2).

4. Procédé selon les revendications 1 à 3, **caractérisé en ce que** la prédispersion est chauffée à une température dans le domaine de 50°C à 70°C par un échangeur de chaleur (8) disposé en amont de la buse d'homogénéisation (2).

5. Procédé selon les revendications 1 à 4, **caractérisé en ce que** la prédispersion est maintenue dans le récipient agité (1) à une température ≤ 50°C.

6. Procédé selon les revendications 1 à 5, **caractérisé en ce que** la dispersion chauffée dans la buse d'homogénéisation (2) est refroidie à une température dans le domaine de 50°C à 70°C par un échangeur de chaleur (9) disposé en aval de la buse d'homogénéisation (2).

7. Procédé selon les revendications 1 à 6, **caractérisé en ce qu'**une pompe à membrane (4) est utilisée comme pompe.

8. Dispositif pour la mise en oeuvre du procédé selon les revendications 1 à 7, à partir d'au moins une buse d'homogénéisation avec un canal d'alimentation et un canal d'évacuation, **caractérisé en ce que** la buse d'homogénéisation consiste en une plaque en céramique dure (11) enfoncée dans un corps en acier (12, 19), avec un alésage (13) de 0,1 mm à 0,5 mm, de préférence de 0,1 mm à 0,2 mm, et **en ce que** le canal d'alimentation (14, 16) conduisant à l'alésage (13) et le canal d'évacuation (15, 18) provenant de l'alésage (13) sont formés de même dans le corps en acier (12, 19).

9. Dispositif selon la revendication 8, **caractérisé en ce que** la plaque en céramique dure (11) consiste en oxyde de zirconium ou en carbure de silicium.

10. Dispositif selon les revendications 8 à 9, **caractérisé en ce que** le corps en acier (19) comporte plusieurs buses d'homogénéisation opposées par paires avec des canaux d'alimentation (16) branchés en parallèle et **en ce que** les canaux d'évacuation (17) débouchent dans un canal collecteur (18) commun dans le corps en acier (19).
